# EUROPÄISCHE PATENTANMELDUNG

(11) **EP 0 821 936 A2**
(43) Veröffentlichungstag der Anmeldung: **04.02.1998**
(21) Anmeldenummer: 97111995.3
(22) Anmeldetag: 15.07.1997
(51) Int. Cl.: A61K 7/32

(54) **Desodorierende Wirkstoffkombinationen auf der Basis von Wollwachssäuren, Partialglyceriden und Arylverbindungen**

(30) Priorität: 01.08.1996 DE 19631004
(71) Anmelder: Beiersdorf Aktiengesellschaft, 20245 Hamburg (DE)
(72) Erfinder: Klier, Manfred, Dr., 21521 Aumühle (DE); Wolf, Florian, Dr., 20251 Hamburg (DE); Liebl, Martina, 22525 Hamburg (DE); Traupe, Bernd, 22457 Hamburg (DE); Hallmann, Marita, 24616 Brokstedt (DE)

(57) **Zusammenfassung**

Wirkstoffkombinationen, bestehend aus
(I) einem natürlichen oder durch Destillation aufbereiteten Gemisch aus Wollwachssäuren
(II) einer oder mehrerer Substanzen, gewählt aus der Gruppe der Monoglycerin-monocarbonsäure-monoester, der Diglycerin-monocarbonsäure-monoester, der Triglycerin-monocarbonsäure-monoester, der Monoglycerin-dicarbonsäure-monoester, der Diglycerin-dicarbonsäure-monoester und der Triglycerin-dicarbonsäure-monoester,
(III) einer oder mehrerer Arylverbindungen.

## Beschreibung

Die vorliegende Erfindung betrifft kosmetische Wirkstoffkombinationen, insbesondere Wirkstoffkombinationen als wirksames Prinzip in kosmetischen Desodorantien.

Kosmetische Desodorantien dienen dazu, Körpergeruch zu beseitigen, der entsteht, wenn der an sich geruchlose frische Schweiß durch Mikroorganismen zersetzt wird. Den handelsüblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Zubereitungen, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichem.
5) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Es wurde überraschenderweise gefunden, und darin liegt die Lösung all dieser Aufgaben, daß Wirkstoffkombinationen, bestehend aus
(I) einem natürlichen oder durch Destillation aufbereiteten Gemisch aus Wollwachssäuren,
(II) einer oder mehrerer Substanzen, gewählt aus der Gruppe der Monoglycerin-monocarbonsäure-monoester, der Diglycerin-monocarbonsäure-monoester, der Triglycerin-monocarbonsäure-monoester, der Monogrycerin-dicarbonsäure-monoester, der Diglycerin-dicarbonsäure-monoester und der Triglycerin-dicarbonsäure-monoester,
(III) einer oder mehrerer Arylverbindungen der allgemeinen Strukturformel 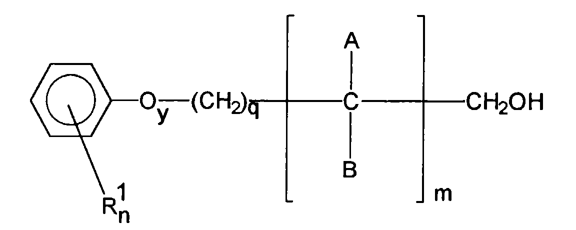 wobei R¹ darstellen kann: H, CH₃, OCH₃, NH₂, und wobei bis zu fünf gleiche oder verschiedene Reste R¹ bzw. beliebige Kombinationen gleicher und verschiedener solcher Reste innerhalb eines Moleküls auftreten können, entsprechend n = 1 - 5, der Index m Werte von 1 - 10 annimmt, der Index q Werte von 0 bis 5 annimmt, der Index y Werte von = oder 1 annimmt, und A und B unabhängig voneinander darstellen: H, OH, CH₃.
bzw. kosmetische Desodorantien, solche Wirkstoffkombinationen enthaltend, den Nachteilen des Standes der Technik abhelfen.

Wollwachs oder Wollfett wird der bei der Rohwollwäsche anfallende fett- bis wachsartige Bestandteil der Rohschafwolle bezeichnet. Das Wollwachs besteht aus aus einem Gemisch von Fettsäureestern höherer Alkohole und aus freien Fettsäuren.

Die Hauptbestandteile der Wollwachssäuren sind
(a) gesättigte unsubstituierte Carbonsäuren, gemäß der Formel

   CH₃―(CH₂)ₙ―CH₂―COOH
(b) α-Hydroxycarbonsäuren, gemäß der Formel 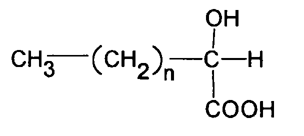 und/oder
(c) ω-Hydroxycarbonsäuren, gemäß der Formel

   HO―CH₂―(CH₂)ₙ―CH₂―COOH
(d) Isocarbonsäuren, gemäß der Formel 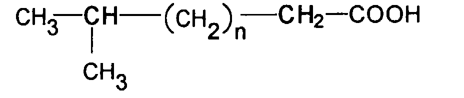
(e) α-Hydroxy-isocarbonsäuren, gemäß der Formel 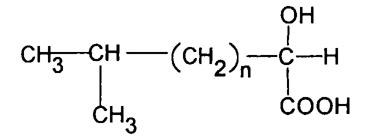 und/oder
(f) ω-Hydroxy-isocarbonsäuren, gemäß der Formel 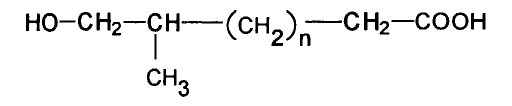
(g) Anteisocarbonsäuren, gemäß der Formel 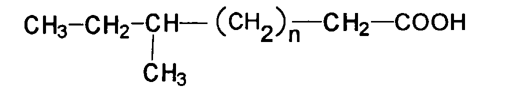
(h) α-Hydroxy-anteisocarbonsäuren, gemäß der Formel 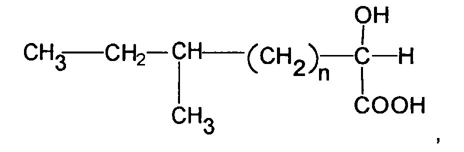
(i) ω-Hydroxy-anteisocarbonsäuren, gemäß der Formel

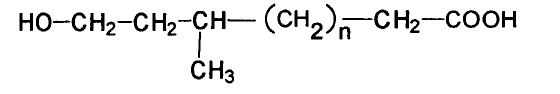

Dabei nimmt n gewöhnlich Werte von 7 - 31 an. Repräsentative Zusammensetzungen der Wollwachssäuren werden z.B. in "Parfümerie und Kosmetik", 59. Jahrgang, Nr.12/78, S.429, 430 sowie im "Lexikon der Hilfsstoffe für Pharmazie, Kosmetik und angrenzende Gebiete" von H.P.Fiedler, 1989, 3. Auflage, Editio Cantor Aulendorf, beschrieben.

Rohwollwachssäuren sind für kosmetische Zwecke nicht geeignet, statt ihrer werden für gewöhnlich destillierte Wollwachssäuren eingesetzt. Dieser Umstand und entsprechende Verfahren zur Raffinierung der Rohwollwachssäuren sind dem Fachmann bekannt.

Besonders vorteilhafte Verfahren zur Aufbereitung von Rohwollwachssäure sind beispielsweise der EP-OS 556 660 zu entnehmen.

Typischerweise bestehen Wollwachssäuren aus ca. 60 % gesättigten, unsubstituierten Carbonsäuren, ca. 30 % α-Hydroxycarbonsäuren und ca. 5 % ω-Hydroxycarbonsäuren, wobei der Rest von ca. 5 % im wesentlichen von den anderen vorgenannten Carbonsäuretypen gebildet wird.

Erfindungsgemäß besonders vorteilhaft ist eine Wollwachssäurefraktion, erhältlich durch Rohwollwachssäure durch Kurzwegdestillation im bei 10⁻¹ bar aus dem Destillationstemperaturintervall von 150 - 200° C. Der Anteil an α-Hydroxycarbonsäuren beträgt dabei ca. 22 - 27 %. Solche Fraktionen zeichnen sich durch folgende kennzeichnende Parameter aus:

| | |
|---|---|
| Tropfpunkt | 50 - 54° C |
| Säurezahl | 166 - 170 |
| Verseifungszahl | 175 - 190 |
| OH-Zahl | 60 - 80 |
| Jodzahl | 7 - 12 |

Zwar ist aus dem Aufsatz "Antimicrobial Factors in Wool Wax" (Australian Journal of Chemistry, 1971, 24, Seiten 153 ff.) bekannt, daß in manchen Wollwachschargen antimikrobielle Faktoren enthalten sind. Ein Hinweis in Richtung der vorliegenden Erfindung findet sich am angegebenen Orte jedoch nicht.

Die erfindungsgemäßen Monoglycerin-mono- bzw. -dicarbonsäure-monoester werden durch die allgemeine Formel 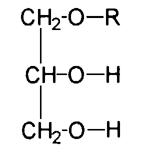 bzw. 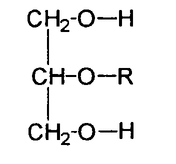 wiedergegeben, wobei R einen verzweigten oder unverzweigten Acylrest mit 6 - 14 Kohlenstoffatomen darstellt. Vorteilhaft wird R gewährt aus der Gruppe der unverzweigten Acylreste. Die diesen Estern zugrundeliegenden Fettsäuren bzw. Monocarbonsäuren sind die

| | | |
|---|---|---|
| Hexansäure | (Capronsäure) | (R = -C(O)-C₅H₁₁), |
| Heptansäure | (Önanthsäure) | (R = -C(O)-C₆H₁₃), |
| Octansäure | (Caprylsäure) | (R = -C(O)-C₇H₁₅), |
| Nonansäure | (Pelargonsäure) | (R = -C(O)-C₈H₁₇), |
| Decansäure | (Caprinsäure) | (R = -C(O)-C₉H₁₉), |
| Undecansäure | | (R = -C(O)-C₁₀H₂₁), |
| Undecensäure | | (R = -C(O)C₁₀H₁₉), |
| Dodecansäure | (Laurinsäure) | (R = -C(O)-C₁₁H₂₃), |
| Tridecansäure | | (R = -C(O)-C₁₂H₂₅), |
| Tetradecansäure | (Myristinsäure) | (R = -C(O)-C₁₃H₂₇). |

Besonders vorteilhaft stellt R den Octanoylrest (Caprylsäurerest) bzw. den Decanoylrest (Caprinsäurerest) dar, wird also also durch die Formeln

R = -C(O)-C₇H₁₅) bzw. R = -C(O)-C₉H₁₉)

repräsentiert.

In dieser Schrift, insbesondere in den Beispielen, wird das Kürzel GMCy für Glycerinmonocaprylat und das Kürzel GMC für Glycerinmonocaprinat verwendet.

Bei den in 1-Position des Glycerins veresterten Glycerinestern ist die 2-Position ein Asymmetriezentrum. Erfindungsgemäß aktiv und gleichermaßen von Vorteil sind die 2S- und die 2R-Konfiguration.

Es hat sich als günstig herausgestellt, racemische Gemische der Stereoisomeren zu verwenden.

In den dermatologischen Zubereitungen beträgt der Gehalt an GMCy und/oder GMC vorteilhaft 0,1 - 10,0 Gew.-%, bevorzugt 0,5 bis 7,5 Gew.-%, besonders bevorzugt 1,5 - 5,0 Gew.-%, jeweils bezogen auf das Gesamtgewicht der jeweiligen Formulierung.

Erfindungsgemäß liegen die Di- bzw. Triglycerineinheiten der erfindungsgemäßen Diglycerin-mono- bzw. -dicarbonsäure-monoester bzw. Triglycerin-mono- bzw. -dicarbonsäure-monoester als lineare, unverzweigte Moleküle, also über die jeweiligen OH-Gruppen in 1- bzw. 3-Stellung veretherte "Monoglycerinmoleküle" vor.

Ein geringer Anteil zyklischer Di- bzw. Triglycerineinheiten sowie über die OH-Gruppen in 2-Stellung veretherte Glycerinmoleküle kann geduldet werden. Es ist jedoch von Vorteil, solche Verunreinigungen so gering wie nur möglich zu halten.

Die erfindungsgemäßen Mono- bzw. Dicarbonsäuremonoester des Diglycerins sind bevorzugt durch folgende Struktur gekennzeichnet (Substitutionspositionen angegeben): 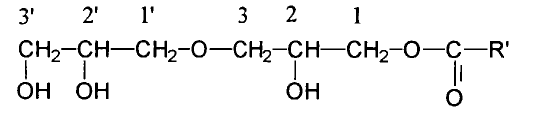 wobei R' einen Kohlenwasserstoffrest, vorteilhaft einen verzweigten oder unverzweigten Alkyl- oder Alkenylrest von 5 bis 17 C-Atomen darstellt.

Die erfindungsgemäßen Mono- bzw. Dicarbonsäuremonoester des Triglycerins sind bevorzugt durch folgende Struktur gekennzeichnet (Substitutionspositionen angegeben): 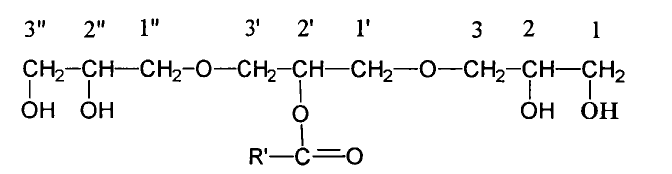 wobei R'' einen Kohlenwasserstoffrest, vorteilhaft einen verzweigten oder unverzweigten Alkyl- oder Alkenylrest Alkylrest von 5 bis 17 C-Atomen darstellt.

Die diesen Estern zugrundeliegenden Monocarbonsäuren sind die

| | | |
|---|---|---|
| Hexansäure | (Capronsäure) | (R' bzw. R''= -C₅H₁₁), |
| Heptansäure | (Önanthsäure) | (R' bzw. R''= -C₆H₁₃), |
| Octansäure | (Caprylsäure) | (R' bzw. R''= -C₇H₁₅), |
| Nonansäure | (Pelargonsäure) | (R' bzw. R''= -C₈H₁₇), |
| Decansäure | (Caprinsäure) | (R' bzw. R''= -C₉H₁₉), |
| Undecansäure | | (R' bzw. R''= -C₁₀H₂₁), |
| 10-Undecensäure | (Undecylensäure) | (R' bzw. R''= -C₁₀H₁₉), |
| Dodecansäure | (Laurinsäure) | (R' bzw. R''= -C₁₁H₂₃), |
| Tridecansäure | | (R' bzw. R''= -C₁₂H₂₅), |
| Tetradecansäure | (Myristinsäure) | (R' bzw. R''= -C₁₃H₂₇), |
| Pentadecansäure | | (R' bzw. R''= -C₁₄H₂₉), |
| Hexadecansäure | (Palmitinsäure) | (R' bzw. R''= -C₁₅H₃₁), |
| Heptadecansäure | (Margarinsäure) | (R' bzw. R''= -C₁₆H₃₃), |
| Octadecansäure | (Stearinsäure) | (R' bzw. R''= -C₁₇H₃₅). |

Besonders günstig werden R' und R'' gewählt aus der Gruppe der unverzweigten Alkylreste mit ungeraden C-Zahlen, insbesondere mit 9, 11 und 13 C-Atomen.

Im allgemeinen sind die Monocarbonsäure-monoester des Diglycerins denen des Triglycerins bevorzugt.

Ganz besonders günstig sind

| | | |
|---|---|---|
| Diglycerinmonocaprinat | (DMC) | R' = 9 |
| Triglycerinmonolaurat | (TML) | R'' = 11 |
| Diglycerinmonolaurat | (DML) | R' = 11 |
| Triglycerinmonomyristat | (TMM) | R'' = 13. |

Als bevorzugter erfindungsgemäßer Monocarbonsäuremonoester des Diglycerins hat sich das Diglycerinmonocaprinat (DMC) erwiesen.

Die erfindungsgemäßen Monocarbonsäuremonoester des Diglycerins liegen bevorzugt in 1-Stellung, die erfindungsgemäßen Monofettsäureester des Triglycerins bevorzugt in 2'-Stellung verestert vor.

Nach einer vorteilhaften Ausführungsform der vorliegenden Erfindung wird ein zusätzlicher Anteil an in anderen Stellen verestertem Di- oder Triglycerin, ebenso wie gegebenenfalls ein Anteil an den verschiedenen Diestern des Di- bzw. Triglycerins verwendet.

Insbesondere vorteilhaft sind solche Monocarbonsäureester, welche nach einem Verfahren erhältlich sind, wie es in der DE-OS 38 18 293 beschrieben wird.

Die den erfindungsgemäßen Estern zugrundeliegenden Dicarbonsäuren werden bevorzugt gewählt aus der Gruppe der α,ω-Alkandicarbonsäuren, insbesondere bevorzugt gewählt aus der Gruppe der α,ω-Alkandicarbonsäuren, so daß die Reste R, R 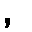 bzw. R 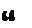 im gegebenen Falle von der generischen Formel

-OOC-(CH₂)ₖ-COOH

beschrieben werden und wobei k Zahlen von 0 bis 8 annehmen kann.

Die Diglycerinester, welche sich durch zwei, und die Triglycerinester, welche sich durch drei Asymmetriezentren auszeichnen, sind in all ihren Konfigurationen erfindungsgemäß wirksam. Die Diglycerinester besitzen vier, die Triglycerinester acht Stereoisomere.

Bei den Diglycerinestern sind die 2- und die 2'-Position Asymmetriezentren. Erfindungsgemäß aktiv und gleichermaßen von Vorteil sind die 2S2'S-, die 2R2'S-, die 2S2'R- und die 2R2'R-Konfiguration.

Bei den Triglycerinestern sind die 2-, die 2' und die 2''-Position Asymmetriezentren. Erfindungsgemäß aktiv und gleichermaßen von Vorteil sind die 2S2'S2''S-, die 2R2'S2''S-, die 2S2'R2''S-, die 2R2'R2''S-, die 2S2'S2''R, die 2R2'S2''R-, die 2S2'R2''R- und die 2R2'R2''R-Konfiguration.

Es hat sich als günstig herausgestellt, racemische Gemische der Stereoisomeren zu verwenden.

Erfindungsgemäß bevorzugte Arylverbindungen werden gewählt aus der Gruppe Phenoxyethanol, Anisalkohol, 2-Methyl-5-phenyl-pentan-1-ol, 2-Methyl-4-phenylbutan-2-ol.

Phenoxyethanol ist gekennzeichnet durch die Strukturformel

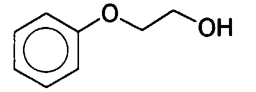

Anisalkohol ist gekennzeichnet durch die Strukturformel

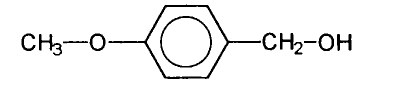

2-Methyl-5-phenyl-pentan-1-ol ist gekennzeichnet durch die Strukturformel

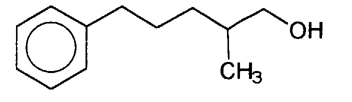

2-Methyl-4-phenylbutan-2-ol ist gekennzeichnet durch die Strukturformel

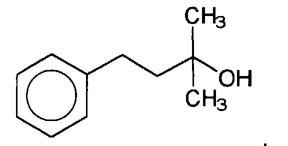

Es ist zwar aus der DE-OS 37 40 186 bekannt, Phenoxyethanol (auch: Phenoxetol) als Bestandteil kosmetischer Desodorantien einzusetzen. Ferner werden am angegebenen Orte auch Kombinationen aus Phenoxyethanol und Glycerinmonolaurat beschrieben. Der Stand der Technik konnte jedoch keinen Hinweis auf die erfindungsgemäßen, synergistisch wirkenden Wirkstoffkombinationen mit Wollwachssäuregemischen geben.

Die erfindungsgemäßen Zubereitungen sind besonders vorteilhaft dadurch gekennzeichnet, daß das Wollwachssäuregemisch in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt.

Die erfindungsgemäßen Zubereitungen sind ferner besonders vorteilhaft dadurch gekennzeichnet, daß der oder die Mond oder Dicarbonsäure-Monoester des Mono-, Di- und/oder Triglycerins in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

Die erfindungsgemäßen Zubereitungen sind weiterhin besonders vorteilhaft dadurch gekennzeichnet, daß die Arylverbindung oder die Arylverbindungen in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

Es ist von Vorteil, den Gehalt an (i) Wollwachssäuregemisch, (ii) Mono-, Di- und/oder Triglycerin-mono- bzw. dicarbonsäure-monoester und (iii) Arylverbindung so zu wählen, daß Verhältnisse (i) : (ii) : (iii) wie 5 : 5 : 1 bis 1 : 20 : 20, insbesondere wie etwa 1 : 1 : 10, ganz besonders vorteilhaft wie etwa 1 : 1 : 5 entstehen.

Es ist insbesondere von Vorteil, den Gehalt an (i) Wollwachssäuren und (ii) Diglycerinmonocaprinat und/oder Glycerinmonocaprylat und/oder Glycerinmonocaprinat so zu wählen, daß Verhältnisse von (i) : (ii) wie 5 : 1 bis 1 : 20 entstehen. Insbesondere günstig im Sinne der vorliegenden Erfindungen sind Verhältnisse von (i) : (ii) wie 5: 1 bis 1 : 5, insbesondere wie etwa 1 : 1, ganz besonders vorteilhaft wie etwa 1 : 3.

Erfindungsgemäße Zubereitungen, die erfindungsgemäßen Wirkstoffkombinationen enthaltend, sind besonders vorteilhaft dadurch gekennzeichnet, daß die erfindungsgemäßen Wirkstoffkombinationen in Konzentrationen von 0,05 - 10,00 Gew.-%, bevorzugt 0,1 - 5,0 Gew.-%, vorliegen, jeweils bezogen auf das Gesamtgewicht der Zubereitungen.

Erfindungsgemäße dermatologische Zubereitungen können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, jedoch auch in Form von aus normalen Flaschen und Behältern auftragbaren W/O- oder O/W-Emulsionen, z.B. Cremes oder Lotionen. Weiterhin können die Zubereitungen vorteilhaft in Form von Tinkturen, Shampoos, Wasch-, Dusch- oder Badezubereitungen oder Pudern vorliegen.

Als übliche kosmetische Trägerstoffe zur Herstellung der erfindungsgemäßen dermatologischen Zubereitungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Als Treibmittel für erfindungsgemäße, aus Aerosolbehältern versprühbare dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindungsgemäßen dermatologischen Zubereitungen, haben sich nichtionogene Typen, wie Polyoxyethylen-Fettalkoholether, z.B. Cetylstearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den erfindungsgemäßen dermatologischen Zubereitungen, deren pH-Wert vorzugsweise z.B. durch übliche Puffergemische auf 4,0 bis 7,5 insbesondere 5,0 bis 6,5, eingestellt wird, Parfüm, Farbstoffe, Antioxidantien, Suspendiermittel, Puffergemische oder andere übliche kosmetische oder dermatologische Grundstoffe beigemischt werden.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofem Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist für die vorliegende Erfindung vorteilhaft, daß der pH-Wert der erfindungsgemäßen dermatologischen Zubereitungen kleiner als 8 ist. pH-Werte, die leicht höher sind als 7, aber kleiner als 7,5 können dabei im allgemeinen toleriert werden. Jedenfalls ist für ein gegebenes Fettsäuregemisch im Einzelfalle durch einfaches Ausprobieren, ohne erfinderisches Dazutun, leicht zu ermitteln, welche exakte obere pH-Grenze zu beachten ist.

Vorteilhaft wird der pH-Wert der erfindungsgemäßen Formulierungen im sauren bis sehr schwach alkalischen Bereich kleiner als 8 eingestellt, bevorzugt von 4,0 - 7,5, besonders bevorzugt von 5,0 - 6,5.

Die jeweils einzusetzenden Mengen an Hilfs- Zusatz- und Trägerstoffen und gegebenenfalls Parfüm können in Abhängigkeit von der Art des jeweiligen Produktes vom Fachmann durch einfaches Ausprobieren leicht ermittelt werden.

Die Herstellung der erfindungsgemäßen dermatologischen Zubereitungen erfolgt, abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von galenischen Formulierungen zu beachten, die dem Fachmann geläufig sind.

Sollen die erfindungsgemäßen Kombinationen in Puder eingearbeitet werden, so können die Suspensionsgrundlagen dafür vorteilhaft gewährt werden aus der Gruppe

Kieselsäuregele (z.B. solche die unter dem Handelsnamen Aerosil® erhältlich sind), Kieselgur, Talkum, modifizierte Stärke, Titandioxid, Seidenpulver, Nylonpulver, Polyethylenpulver und verwandten Stoffen.

Es folgen vorteilhafte Ausführungsbeispiele der vorliegenden Erfindung. Die Mengenangaben beziehen sich stets auf Gewichts-%, wenn nichts Anderes angegeben wird. In den Beispielen bedeutet der Begriff "WWS" eine Wollwachssäurefraktion, welche gewonnen wurde aus Rohwollwachssäure durch Kurzwegdestillation im bei 10⁻¹ bar aus dem Destillationstemperaturintervall von 150 - 200° C. Der Anteil an α-Hydroxycarbonsäuren beträgt dabei ca. 22 - 27 %.

| **Beispiel 1** W/O-Crème | |
|---|---|
| | Gew.-% |
| Paraffinöl | 10,00 |
| Ozokerit | 4,00 |
| Vaseline | 4,00 |
| pflanzliches Öl | 10,00 |
| Wollwachsalkohol | 2,00 |
| Aluminiumstearat | 0,40 |
| WWS | 2,00 |
| GMC | 0,50 |
| Phenoxyethanol | 0,20 |
| Parfum, Konservierungsstoffe | q.s. |
| Wasser, VES | ad 100,00 |
| pH: | ad 5,5 - 6,0 |

| **Beispiel 2** W/O-Lotion | |
|---|---|
| | Gew.-% |
| Paraffinöl | 25,00 |
| Siliconöl | 2,00 |
| Ceresin | 1,50 |
| Wollwachsalkohol | 0,50 |
| Glucosesesquiisostearat | 2,50 |
| WWS | 2,00 |
| DML | 0,40 |
| Anisalkohol | 0,10 |
| Parfum, Konservierungsstoffe | q.s. |
| Wasser, VES | ad 100,00 |
| pH: | ad 5,5 - 6,0 |

| **Beispiel 3** O/W-Crème | |
|---|---|
| | Gew.-% |
| Pflanzliches Öl | 10,00 |
| Cetylalkohol | 2,00 |
| Glycerinmonostearat | 1,50 |
| PEG-30-Glycerylstearat | 2,00 |
| Glycerin | 3,00 |
| Isopropylpalmitat | 5,00 |
| Carbopol 980 (neutralisiert) | 0,30 |
| WWS | 2,50 |
| DMC | 0,80 |
| Rosaphen | 0,30 |
| Parfum, Konservierungsstoffe | q.s. |
| Wasser, VES | ad 100,00 |
| pH: | ad 5,5 - 6,0 |

| **Beispiel 4** Hautöl | |
|---|---|
| | Gew.-% |
| Cetylpalmitat | 3,00 |
| C₁₂₋₁₅-Alkylbenzoat | 2,00 |
| Polyisobuten | 10,00 |
| Squalan | 2,00 |
| WWS | 1,00 |
| GML | 0,40 |
| Muguet-Alkohol | 0,10 |
| Parfum, Konservierungsstoffe | q.s. |
| Paraffinöl | ad 100,00 |

| **Beispiel 5** Badeöl | |
|---|---|
| | Gew.-% |
| Paraffinöl | 20,00 |
| PEG-40-hydriertes Rizinusöl | 5,00 |
| WWS | 1,00 |
| TMM | 0,50 |
| Phenoxyethanol | 0,20 |
| Parfum, Konservierungsstoffe | q.s. |
| Sojaöl | ad 100,00 |

| **Beispiel 6** Pflegemaske | |
|---|---|
| | Gew.-% |
| PEG-50 Lanolin | 0,50 |
| Glycerylstearat | 2,00 |
| Sonnenblumenkernöl | 3,00 |
| Bentonit | 8,00 |
| Kaolin | 35,00 |
| Zinkoxid | 5,00 |
| WWS | 0,50 |
| GMCy | 0,30 |
| Anisalkohol | 0,10 |
| Parfum, Konservierungsstoffe | q.s. |
| Wasser, VES | ad 100,00 |
| pH: | ad 5,5 - 6,0 |

| **Beispiel 7** Liposomenhaltiges Gel | |
|---|---|
| | Gew.-% |
| Lecithin | 6,00 |
| Pflanzliches Öl | 12,50 |
| Hydrolysiertes Kollagen | 2,00 |
| Xanthan Gum | 1,40 |
| Butylenglycol | 3,00 |
| WWS | 0,60 |
| DMC | 0,50 |
| Phenyl-dimethylethyl-carbinol | 0,10 |
| Parfum, Konservierungsstoffe | q.s. |
| Wasser, VES | ad 100,00 |
| pH: | ad 5,5 - 6,0 |

| **Beispiel 8** Duschpräparat mit Rückfetter | |
|---|---|
| | Gew.-% |
| Cocoamidodiacetat | 10,00 |
| Natriumlaurylsulfat | 25,00 |
| Kalium Cocyl Hydrolysiertes Kollagen | 5,00 |
| Macadamianußöl | 5,00 |
| Natriumchlorid | 0,60 |
| WWS | 1,00 |
| DML | 0,50 |
| Anisalkohol | 0,30 |
| Parfum, Konservierungsstoffe | q.s. |
| Wasser, VES | ad 100,00 |
| pH: | ad 5,5 - 6,0 |

| **Beispiel 9** Seifenstück | |
|---|---|
| | Gew.-% |
| Na-Salz aus Talgfettsäuren | 60,00 |
| Na-Salz aus Kokosöl | 28,00 |
| Natriumchlorid | 0,50 |
| WWS, Natriumsalz | 5,00 |
| DML | 1,00 |
| Phenoxyethanol | 0,40 |
| Parfum, Konservierungsstoffe | q.s. |
| Wasser, VES | ad 100,00 |
| pH: | ad 5,5 - 6,0 |

| **Beispiel 10** Pflegeshampoo | |
|---|---|
| | Gew.-% |
| Natriumlaurylsulfat | 34,00 |
| Dinatriumlaurylsulfosuccinat | 6,00 |
| Cocoamidopropylbetain | 10,00 |
| Glycoldistearat | 5,00 |
| WWS | 2,00 |
| GML | 1,00 |
| Pelargol | 0,40 |
| Parfum, Konsevierungsstoffe | q.s. |
| Wasser, VES | ad 100,00 |
| pH: | ad 5,5 - 6,0 |

| **Beispiel 11** Rasierschaum | |
|---|---|
| | Gew.-% |
| Stearinsäure | 7,00 |
| Natriumlaurylsulfat | 3,00 |
| Stearylalkohol | 1,00 |
| Glycerin | 5,00 |
| Triethanolamin | 3,60 |
| WWS | 0,60 |
| DML | 0,30 |
| Phenyl-dimethylethyl-carbinol | 0,10 |
| Parfum, Konservierungsstoffe | q.s. |
| Wasser, VES | ad 100,00 |
| pH: | ad 5,5 - 6,0 |

| **Beispiel 12** Fußcrème | |
|---|---|
| | Gew.-% |
| Soluan 5 | 2,00 |
| Methylsalicylat | 5,00 |
| Caprylic/Capric Triglyceride | 10,00 |
| Stearinsäure | 5,00 |
| Cetylalkohol | 1,00 |
| Glycerin | 2,00 |
| Dimethicon | 1,00 |
| Carbopol 984 | 0,50 |
| Triethanolamin | 1,50 |
| WWS | 2,00 |
| GML | 1,50 |
| Pelargol | 0,60 |
| Parfum, Konservierungsstoffe | q.s. |
| Wasser, VES | ad 100,00 |
| pH: | ad 5,5 - 6,0 |

| **Beispiel 13** Pumpspray | |
|---|---|
| | Gew.-% |
| PEG-40-Hydriertes Rizinusöl | 2,00 |
| Glycerin | 1,00 |
| WWS | 0,40 |
| DML | 0,40 |
| Anisalkohol | 0,20 |
| Parfum, Konservierungsstoffe | q.s. |
| Wasser, VES | ad 100,00 |
| pH: | ad 5,5 - 6,0 |

| **Beispiel 14** Roll-on-Gel | |
|---|---|
| | Gew.-% |
| 1,3-Butylenglycol | 2,00 |
| PEG-40-Hydriertes Rizinusöl | 2,00 |
| Hydroxyethylcellulose | 0,50 |
| WWS | 2,00 |
| DML | 0,70 |
| Anisalkohol | 0,10 |
| Parfum, Konservierungsstoffe | q.s. |
| Wasser, VES | ad 100,00 |
| pH: | ad 5,5 - 6,0 |

| **Beispiel 15** Roll-on-Emulsion | |
|---|---|
| | Gew.-% |
| Tricetearethphosphat | 0,30 |
| Octyldodecanol | 2,00 |
| C₁₂₋₁₅-Alkylbenzoat | 2,00 |
| C₁₀₋₃₀-Alkylacrylat | 0,15 |
| WWS | 0,70 |
| DML | 0,40 |
| Anisalkohol | 0,20 |
| Parfum, Konservierungsstoffe | q.s. |
| Wasser, VES | ad 100,00 |
| pH: | ad 5,5 - 6,0 |

| **Beispiel 16** Wachsstift | |
|---|---|
| | Gew.-% |
| Hydriertes Rizinusöl | 5,00 |
| Bienenwachs | 6,00 |
| Ceresin | 30,00 |
| C₁₂₋₁₅-Alkylbenzoat | 17,00 |
| WWS | 2,50 |
| TMM | 0,80 |
| Anisalkohol | 0,40 |
| Parfum, Konservierungsstoffe | q.s. |
| Octyldodecanol | ad 100,00 |

## Patentansprüche

1. Wirkstoffkombinationen, bestehend aus
(I) einem natürlichen oder durch Destillation aufbereiteten Gemisch aus Wollwachssäuren
(II) einer oder mehrerer Substanzen, gewählt aus der Gruppe der Monoglycerin-monocarbonsäure-monoester, der Diglycerin-monocarbonsäure-monoester, der Triglycerin-monocarbonsäure-monoester, der Monoglycerin-dicarbonsäure-monoester, der Diglycerin-dicarbonsäure-monoester und der Triglycerin-dicarbonsäure-monoester,
(III) einer oder mehrerer Arylverbindungen der allgemeinen Strukturformel 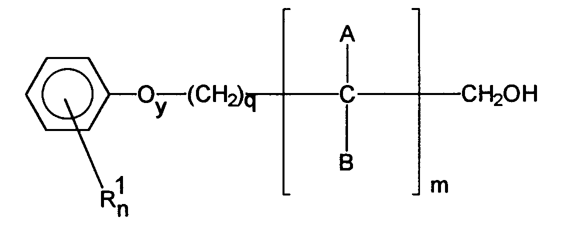 wobei R¹ darstellen kann: H, CH₃, OCH₃, NH₂, und wobei bis zu fünf gleiche oder verschiedene Reste R¹ bzw. beliebige Kombinationen gleicher und verschiedener solcher Reste innerhalb eines Moleküls auftreten können, entsprechend n = 1 - 5, der Index m Werte von 1 - 10 annimmt, der Index q Werte von 0 bis 5 annimmt, der Index y Werte von = oder 1 annimmt, und A und B unabhängig voneinander darstellen: H, OH, CH_{3.}.

2. Wirkstoffkombinationen nach Anspruch 1, dadurch gekennzeichnet, daß das Wollwachssäuregemisch erhältlich ist aus Rohwollwachssäure durch Kurzwegdestillation im Vakuum bei 10⁻¹ bar aus dem Destillationstemperaturintervall von 150 - 200° C.

3. Wirkstoffkombinationen nach Anspruch 1, dadurch gekennzeichnet, daß das durch Destillation erhältliche Wollwachssäuregemisch sich durch folgende Parameter auszeichnet:
| | |
|---|---|
| Tropfpunkt | 50 - 54° C |
| Säurezahl | 166 - 170 |
| Verseifungszahl | 175 - 190 |
| OH-Zahl | 60 - 80 |
| Jodzahl | 7 - 12 |

4. Wirkstoffkombinationen nach Anspruch 1, dadurch gekennzeichnet, daß die Substanz oder die Substanzen der Gruppe der Monoglycerin-monocarbonsäure-monoester, der Diglycerin-monocarbonsäure-monoester und der Triglycerin-monocarbonsäure-monoester gewährt wird aus der Gruppe Glycerinmonocaprylat, Glycerinmonocaprinat, Diglycerinmonocaprinat, Triglycerinmonolaurat, Diglycerinmonolaurat, Triglycerinmonomyristat.

5. Wirkstoffkombinationen nach Anspruch 1, dadurch gekennzeichnet, daß als Arylverbindung das Phenoxyethanol gewählt wird.
